# EUROPEAN PATENT APPLICATION

(11) **EP 2 392 658 A2**
(43) Date of publication of application: **07.12.2011**
(21) Application number: 11003894.0
(22) Date of filing: 11.05.2011
(51) Int. Cl.: C12N 15/10, A61K 8/73, B01J 13/00

(54) **Centrifugation method**

(30) Priority: 13.05.2010 JP 2010111134
(71) Applicant: TAKARA BIO INC., Otsu-shi, Shiga (JP)
(72) Inventor: Usui, Kanako, Otsu-shi, Shiga (JP); Kishida, Sayuri, Otsu-shi, Shiga (JP); Tanaka, Tomomi, Otsu-shi, Shiga (JP); Mukai, Hiroyuki, Otsu-shi, Shiga (JP)
(74) Representative: Vossius & Partner

(57) **Abstract**

A centrifugation method for separating a high specific gravity substance and a low specific gravity substance from a mixture thereof, which prevents the contamination of the high specific gravity substance in collecting the low specific gravity substance after a separation operation, is provided. The centrifugation method includes centrifuging a mixture comprising a high specific gravity substance, a low specific gravity substance, and at least one of a thermoreversible gel and a dissolved gelling agent capable of forming a thermoreversible gel, in which the thermoreversible gel or the dissolved gelling agent forms a gel layer which separates the low specific gravity substance and the high specific gravity substance. The centrifugation is preferably carried out at a temperature equal to or lower than the gelation temperature of the gelling agent.

## Description

### FOREIGN PRIORITY

This application is based on Japanese Patent Application No. 2010-111134, filed on May 13, 2010, the entire contents thereof being hereby incorporated by reference.

### FIELD OF THE INVENTION

The present invention relates to a centrifugation method for separating a high specific gravity substance and a low specific gravity substance from a mixture thereof.

### BACKGROUND

The solid-liquid separation method, which is one of the methods for separating a high specific gravity substance and a low specific gravity substance from a mixture thereof, can be roughly divided into a filtration separation method and a precipitation separation method. The filtration separation method is a method in which a solid substance and a liquid substance are separated by a filtration operation using a filtering material such as a filter, a screen, a porous membrane and the like, and using a natural gravity, a compression force, a decompression force, a centrifugal force and the like as the driving force. The precipitation separation method is a method in which a solid substance in a mixture is precipitated by the natural precipitation or centrifugal force, and thereby separated from a liquid substance in the supernatant, and a method which uses centrifugal force is called a centrifugation method.

In the precipitation separation methods such as the centrifugation method, there is a case in which a precipitate is contaminated into a supernatant when the supernatant is separated from the precipitate. Particularly, when an upper portion of the precipitate is a shape of slurry, it is difficult to avoid contamination of the precipitate into the supernatant. As a means for dissolving this problem, a method using a precipitation auxiliary agent is known (for example, Patent Document 1). However, it is necessary to select a proper precipitation auxiliary agent depending on the kind of the solid substance, and further, there are cases in which a proper precipitation auxiliary agent is not known, so that this method lacks in flexibility.

In addition, though the centrifugation method has also been used to separate a mixture of liquids having different specific gravities (liquid-liquid separation). However, even in the case of the centrifugation-aided liquid-liquid separation, there is a case in which a lower layer substance is contaminated when fractionating the layer formed in the outermost layer after centrifugation.
[Patent Document 1] International Publication No. WO2005/063979

### BRIEF SUMMARY

One of the objects of the present invention is to provide a centrifugation method which can dissolve the problems as mentioned above.

The present inventors have conducted intensive studies, and as a result found that it is possible to prevent contamination of a precipitate into a supernatant by using a thermoreversible gel or a dissolved gelling agent capable of forming a thermoreversible gel in solid-liquid separation by a centrifugation method. In addition, the present inventors have found that it is possible to prevent contamination of a high specific gravity substance into a low specific gravity substance by using a thermoreversible gel or a dissolved gelling agent capable of forming a thermoreversible gel in liquid-liquid separation by a centrifugation method, thereby accomplishing the present invention.

That is, the present invention includes the following embodiments.
[1] A centrifugation method for separating a high specific gravity substance and a low specific gravity substance, which comprises:
   centrifuging a mixture comprising a high specific gravity substance, a low specific gravity substance, and at least one of a thermoreversible gel and a dissolved gelling agent capable of forming a thermoreversible gel,
   wherein the thermoreversible gel or the dissolved gelling agent forms a gel layer which separates the low specific gravity substance and the high specific gravity substance.
[2] The centrifugation method of [1], wherein the centrifugation is carried out at a temperature equal to or lower than the gelation temperature of the gelling agent.
[3] The centrifugation method of [1] or [2], wherein the mixture comprises a solid and a liquid.
[4] The centrifugation method of [1], wherein the mixture is obtained by:
   (a) mixing the high specific gravity substance, the low specific gravity substance and the gelling agent, or mixing a composition comprising the high specific gravity substance and the low specific gravity substance with the gelling agent; and
   (b) heating the mixture comprising the high specific gravity substance, the low specific gravity substance and the gelling agent to dissolve the gelling agent.
[5] The centrifugation method of [1], wherein the mixture is obtained by:
   (A) obtaining a solution in which the gelling agent is dissolved; and
   (B) mixing the solution with the high specific gravity substance and the low specific gravity substance, or with a composition comprising the high specific gravity substance and the low specific gravity substance.
[6] The centrifugation method of [1] to [5], wherein the mixture comprises an ion exchange resin.
[7] The centrifugation method of [6], wherein the ion exchange resin is a chelate resin.
[8] The centrifugation method of [1] or [2], wherein the mixture comprises an aqueous liquid and an organic liquid.
[9] The centrifugation method of [8], wherein the organic liquid is at least one selected from the group consisting of phenol, chloroform and isoamyl alcohol.
[10] The centrifugation method of [8] or [9], wherein the aqueous liquid comprises a nucleic acid.
[11] The centrifugation method of [1] to [10], wherein the gelling agent is a hydrophilic gelling agent.
[12] The centrifugation method of [11], wherein the hydrophilic gelling agent is agarose.
[13] The centrifugation method of [1] to [12], wherein the gelling agent is partly dissolved in the mixture.
[14] The centrifugation method of [1] to [13], which further comprises collecting the low specific gravity substance.
[15] A composition comprising a hydrophilic gelling agent capable of forming a thermoreversible gel and an ion exchange resin.

According to the exemplary embodiments of the present invention, it is possible to easily prevent contamination of a precipitate into supernatant, which was a problem in fractionating the supernatant by the precipitation separation method. In addition, it is possible to easily prevent contamination of a high specific gravity substance into a low specific gravity substance, which was a problem in the liquid-liquid separation making use of centrifugation.

### BRIEF DESCRIPTION OF THE DRAWINGS

Fig. 1A is a figure showing an example of the solid-liquid separation method of an exemplary embodiment of the present invention.
Fig. 1B is a figure showing an example of the solid-liquid separation method of an exemplary embodiment of the present invention.

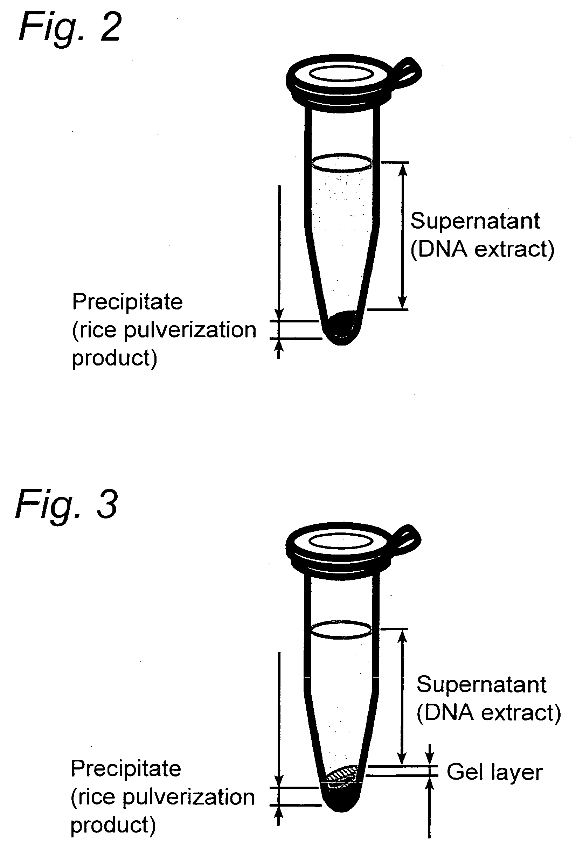

Fig. 2 is a figure showing a condition after centrifugation for the DNA extraction from rice by a conventional method.
Fig. 3 is a figure showing a condition after centrifugation for the DNA extraction from rice by the method of an exemplary embodiment of the present invention.

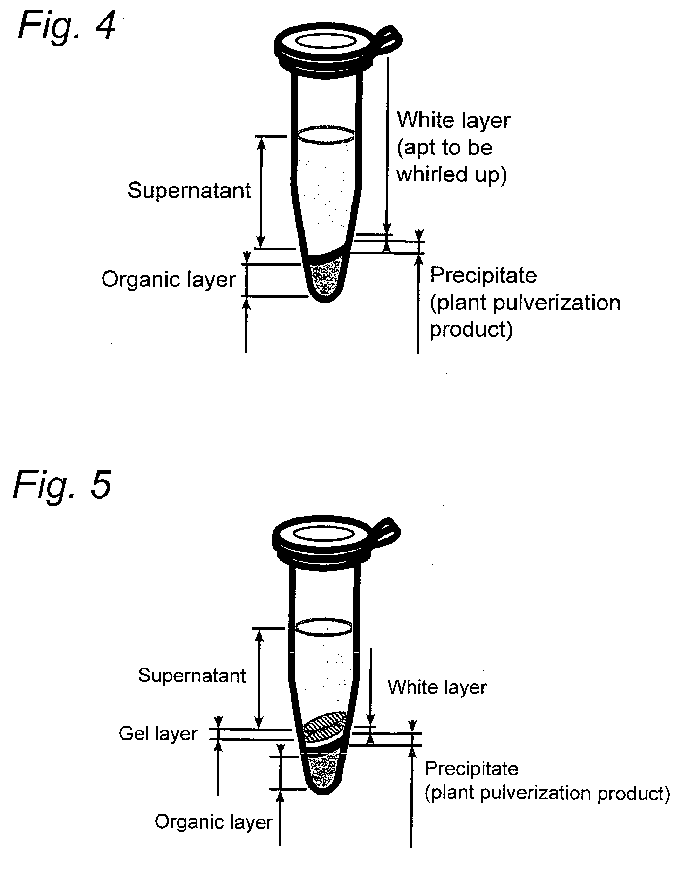

Fig. 4 is a figure showing a condition after centrifugation for the DNA extraction from a plant by the conventional method.
Fig. 5 is a figure showing a condition after centrifugation for the DNA extraction from a plant by the method of an exemplary embodiment of the present invention.

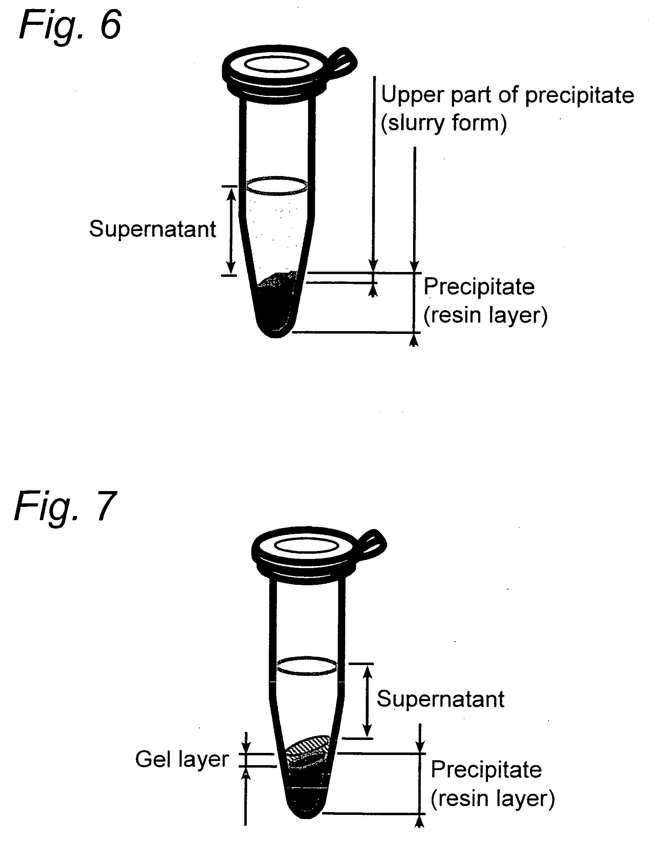

Fig. 6 is a figure showing a condition after centrifugation for the DNA extraction from a formalin-fixed paraffin-embedded tissue section by the conventional method.
Fig. 7 is a figure showing a condition after centrifugation for the DNA extraction from a formalin-fixed paraffin-embedded tissue section by the method of an exemplary embodiment of the present invention.
Fig. 8 is a figure showing a result of agarose gel electrophoresis of the PCR products obtained by using the 164 bp region in rat CCND gene as the target sequence in Example 6.
   The lanes M indicate a 100 bp DNA ladder marker. The lane 1 indicates the negative control. The lane 2 indicates the positive control. The lanes 3 and 6 indicate the PCR products using the supernatants, which were collected using the samples without the gelling agent by paying a considerable attention in order to avoid contamination of the resin layer, as templates for the PCR at an amount of 2.5 µl and µl, respectively. The lanes 4 and 7 indicate the PCR products using the supernatants, which were collected using the samples without the gelling agent by intentionally contaminating the resin layer in a small amount, as templates for the PCR at an amount of 2.5 µl and 1µl, respectively. The lanes 5 and 8 indicate the PCR products using the supernatants, which were collected using the samples with the gelling agent, as templates for the PCR at an amount of 2.5 µl and 1 µl, respectively.

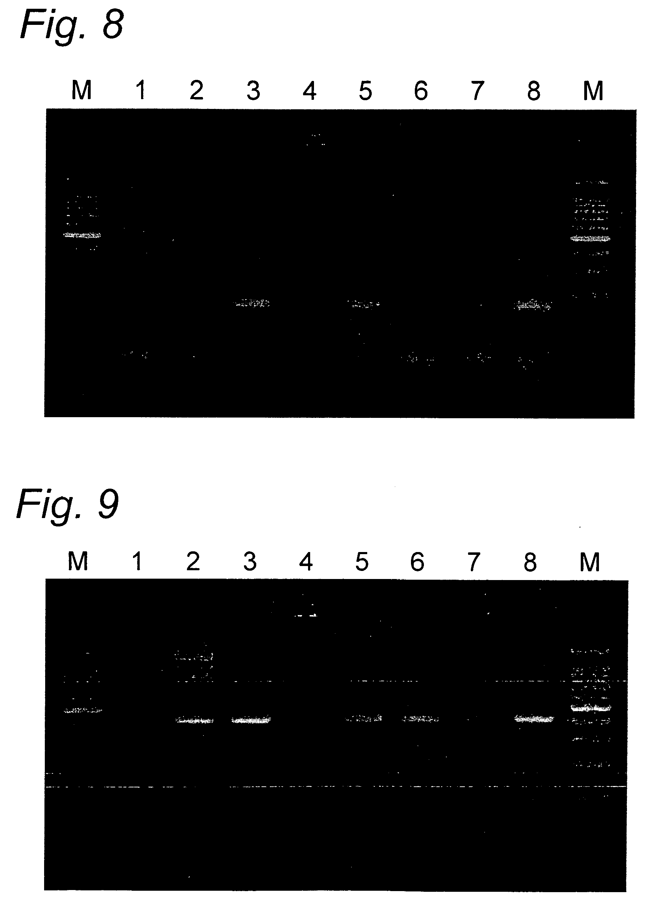

Fig. 9 is a figure showing a result of agarose gel electrophoresis of the PCR using the 416 bp region in rat CCND gene as the target sequence in Example 6.
   The lanes M indicate a 100 bp DNA ladder marker. The lane 1 indicates the negative control. The lane 2 indicates the positive control. The lanes 3 and 6 indicate the PCR products using the supernatants, which were collected using the samples without the gelling agent by paying a considerable attention in order to avoid contamination of the resin layer, as templates for the PCR at an amount of 2.5 µl and 1µl, respectively. The lanes 4 and 7 indicate the PCR products using the supernatants, which were collected using the samples without the gelling agent by intentionally contaminating the resin layer in a small amount, as templates for the PCR at an amount of 2.5 µl and µl, respectively. The lanes 5 and 8 indicate the PCR products using the supernatants, which were collected using the samples with the gelling agent, as templates for the PCR at an amount of 2.5 µl and 1µl, respectively.

### DETAILED DESCRIPTION OF EXEMPLARY EMBODIMENTS

The following describes the exemplary embodiments of the present invention in detail. In this connection, the "%" in the following descriptions means "(weight/volume) %" unless otherwise noted.

The centrifugation method of the exemplary embodiments of the present invention is a method for separating a high specific gravity substance and a low specific gravity substance by centrifugation, in which a thermoreversible gel layer is formed to separate the low specific gravity substance and the high specific gravity substance, by carrying out centrifugation in the presence of a thermoreversible gel and/or a dissolved gelling agent capable of forming a thermoreversible gel. The centrifugation method of the exemplary embodiments of the present invention can be applied to both of the solid-liquid separation method and liquid-liquid separation method.

The high specific gravity substance and the low specific gravity substance of the exemplary embodiments of the present invention are not specifically limited, as long as the high specific gravity substance has a specific gravity higher than the specific gravity of the low specific gravity substance.

The low specific gravity substance may be a substance, a composition, or a mixture thereof, which contains a component that is desired to be separated from other components, using the centrifugation method of the present invention. The low specific gravity substance preferably is a liquid substance, and more preferably is an aqueous liquid substance.

The high specific gravity substance may be a substance, a composition, or a mixture thereof, which contains a component that is desired to be removed, using the centrifugation method of the present invention. The high specific gravity substance may be a solid substance, a liquid substance, or a mixture thereof. When the high specific gravity substance is a liquid substance, it may be an aqueous liquid or an organic liquid, and preferably is an organic liquid.

The gelling agent capable of forming a thermoreversible gel (which is also simply referred to as a gelling agent) used in the exemplary embodiments of the present invention is not particularly limited, as long as it is a gelling agent which can cause a thermoreversible sol-gel transition, and it may be a hydrophilic gelling agent or a lipophilic gelling agent. Preferable examples thereof include a gelling agent which forms a gel layer at the desired position, when a dissolved product of the gelling agent is prepared and centrifugation is carried out at a temperature equal to or lower than its gelation temperature. Though it is not particularly limited, a hydrophilic gelling agent can be suitably used, when the low specific gravity substance is an aqueous liquid substance. As the hydrophilic gelling agent capable of forming a thermoreversible gel, agarose, carrageenan, gellan gum, tamarind seed gum, furcelleran, pectin and gelatin can be exemplified, and agarose can be exemplified most suitably. A composition which contains the aforementioned gelling agent, such as agar that contains agarose as the main component, may be used as the gelling agent.

The thermoreversible gel in the exemplary embodiments of the present invention is not particularly limited, as long as it is a thermoreversible sol-gel transition. The thermoreversible gel may be prepared from the above-described gelling agent.

Amount of the thermoreversible gel and/or the gelling agent used can be optionally determined by verifying conditions of the gel layer formation after centrifugation. When it is desirable to form a thick gel layer and thereby securely prevent contamination of the high specific gravity substance into the low specific gravity substance after centrifugation, concentration of the thermoreversible gel and/or the gelling agent in the mixture containing the high specific gravity substance, the low specific gravity substance, and the thermoreversible gel and/or the gelling agent, may be set to a higher level. When it is desirable to increase collection yield of low specific gravity substance, concentration of the thermoreversible gel and/or the gelling agent in the mixture may be set to a lower level, within a range that the gel layer is formed after centrifugation. Though it is not particularly limited, when agarose is used as the gelling agent, concentration of agarose in the mixture containing the high specific gravity substance, the low specific gravity substance, and agarose is suitably from 0.05% to 1.0%, more suitably from 0.075% to 0.75%, further suitably from 0.1% to 0.3%.

A condition for centrifugation is not particularly limited with the proviso that it is a condition that the low specific gravity substance forms a upper layer, and that a gel layer is formed to separate the low specific gravity substance and the high specific gravity substance after centrifugation. The condition for centrifugation can be decided by examining it in response to the kind of the thermoreversible gel and/or the gelling agent, concentration of the thermoreversible gel and/or the gelling agent in the mixture, kind of the liquid substance to be used as the solvent of the thermoreversible gel and/or the gelling agent, and the like. Though it is not particularly limited, when agarose is used as the gelling agent, centrifugal force is preferably from 5000 G to 30000 G, more preferably from 10000 G to 20000 G, and further preferably 12000 G to 18000 G. Also, the centrifugation time is preferably from 1 minute to 1 hour, more preferably from 3 minutes to 30 minutes, and further preferably from 5 minutes to 20 minutes. The temperature for centrifugation may be a temperature equal to or lower than the gelation temperature of the gelling agent. Though it is not particularly limited, when Agarose LO3 "TaKaRa" (manufactured by TAKARA BIO INC.) is used as the gelling agent, the temperature for centrifugation is preferably below 37°C , more preferably below 34°C, and further preferably below 10°C.

The thermoreversible gel and/or the dissolved gelling agent form a gel layer after centrifugation, which separates the low specific gravity substance and the high specific gravity substance. The term "separate the low specific gravity substance and the high specific gravity substance" means that the gel layer is formed as a layer (a lower layer) which separates the layer of the low specific gravity substance (an upper layer) and the high specific gravity substance which may form a layer. In this connection, the term "lower layer" according to this specification means a layer formed at a position that is lower than the liquid layer which is formed as the upper layer by centrifugation. A layer other than the aforementioned upper layer (intermediate layer) that can be distinguished from the lowermost layer is included in the meaning of the term "lower layer" according to this specification. When three or more layers are formed after centrifugation, the gel layer may be formed at any position of the two or more of the lower layers within such a range that contamination of the high specific gravity substance into the low specific gravity substance can be prevented. When the high specific gravity substance is precipitated after centrifugation, and the precipitate does not form a layer, there is a case in which the gel layer is formed in the form of including the precipitate. In addition, for example, when a substance having a specific gravity higher than that of a precipitate is present in the mixture, the precipitate or a layer of the precipitate is not always positioned at the lowermost layer after centrifugation.

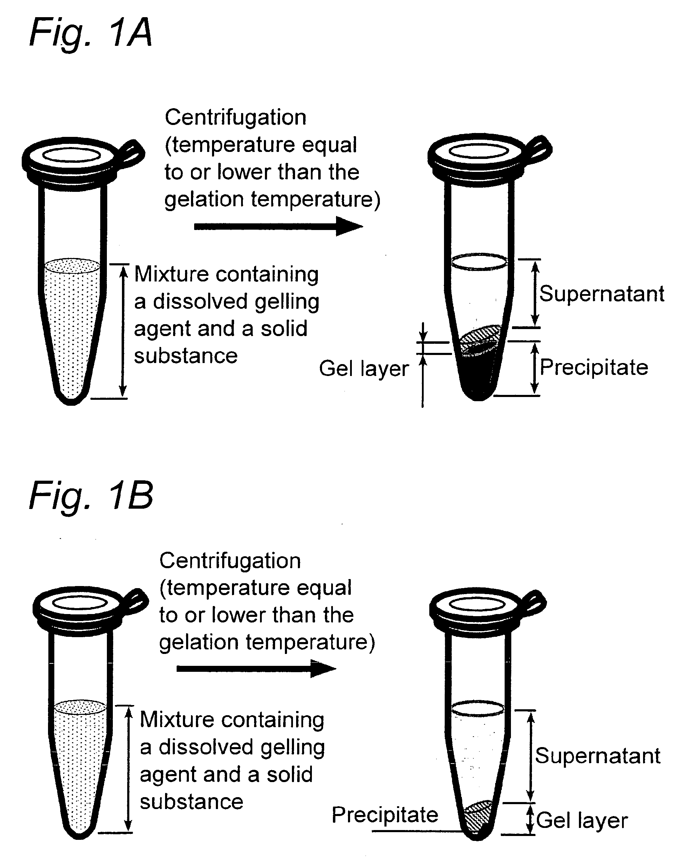

An embodiment of the centrifugation method of the present invention is a solid-liquid separation method. The solid-liquid separation method contains a step of centrifuging a mixture containing a solid (a high specific gravity substance), a liquid (a low specific gravity substance), and a thermoreversible gel and/or a dissolved gelling agent capable of forming a thermoreversible gel, in which the thermoreversible gel and/or the dissolved gelling agent forms a gel layer as a lower layer which is separated from the liquid layer as an upper layer. Centrifugation is preferably carried out at a temperature equal to or lower than the gelation temperature of the gelling agent. The method may further include a step of collecting the lower specific gravity substance. Figures schematically showing examples of the solid-liquid separation method of the exemplary embodiments of the present invention are shown in Fig. 1A and Fig. 1B. Fig. 1B shows an embodiment in which the precipitate does not form a layer and the gel layer is formed in the form of including the precipitate.

According to the solid-liquid separation method of the exemplary embodiments of the present invention, after obtaining the mixture including the high specific gravity substance, the low specific gravity substance and the dissolved gelling agent for forming a thermoreversible gel, entire portion of the aforementioned mixture or a part thereof may be gelatinized at a temperature equal to or lower than the gelation temperature of the gelling agent and then the gelatinized mixture may be subjected to centrifugation.

When a step for collecting the low specific gravity substance (the liquid) is included in the solid-liquid separation method of the exemplary embodiments of the present invention, there is no particular limitation to the collection method. Even when it is difficult to prevent contamination of the precipitate into the liquid by the conventional precipitation separation method because upper part of the precipitate becomes a slurry form, contamination of the precipitate into the liquid.can be prevented by the solid-liquid separation method of the exemplary embodiments of the present invention so that the liquid can also be collected, for example by decantation.

The method for obtaining the mixture comprising the high specific gravity substance, the low specific substance and the thermoreversible gel and/or the dissolved gelling agent capable of forming a thermoreversible gel is not particularly limited. An embodiment of the solid-liquid separation method of the present invention is a method in which the mixture is obtained by (a) mixing the high specific gravity substance, the low specific gravity substance and the gelling agent, or mixing a composition comprising the high specific gravity substance and the low specific gravity substance with the gelling agent, and (b) heating the mixture the high specific gravity substance, the low specific gravity substance and the gelling agent to dissolve the gelling agent.

For example, when it is the purpose to remove an unnecessary substance from a liquid as a sample using a solid substance (e.g., the unnecessary substance is removed by attaching it to the solid substance), removal of the unnecessary substance can be carried out by preparing a mixture of the gelling agent and solid substance in advance and then carrying out the centrifugation method of the exemplary embodiments of the present invention by adding this to the liquid. In that case, the mixture of the gelling agent and the solid substance may be used in the form of a suspension in order to facilitate its addition to the sample. Also, when it is the purpose to extract a desired substance from a solid as a sample, extraction of the intended substance can be carried out by preparing a mixture containing a liquid to be used as an extraction solvent and the gelling agent in advance and then carrying out the centrifugation method of the exemplary embodiments of the present invention by adding this to the sample. In addition, when it is desirable to simultaneously carry out removal of an unnecessary substance from a sample (by using the solid substance) and extraction of a desired substance (by using an extraction solvent), the method of the exemplary embodiments of the present invention can be carried out by preparing a mixture containing the gelling agent, the solid substance and the extraction solvent in advance and then adding this to the sample.

Though shape of the aforementioned gelling agent is not particularly limited, there may be exemplified powder or micro-gel which can be easily dissolved by the subsequent heating. The micro-gel is bulk materials of fine gels and means a gel having fluidity as if it is an aqueous solution, which can be obtained by applying an agitation or the like force to a solution prepared by dissolving the gelling agent, at the time of cooling to effect destruction of gel formation. By the use of the gelling agent in the form of micro-gel, dispersion of the gelling agent in the mixing treated product becomes easy. The micro-gel may be prepared in accordance with the method described in a conventionally known reference (for example, Japanese Patent No. 2513506).

As the aforementioned solid substance for removing the unnecessary substance, though it is not particularly limited, an ion exchange resin, activated carbon, silica gel and an affinity resin can be exemplified. As the ion exchange resin, for example, an anionic ion exchange resin, a cationic exchange resin and a chelate resin can be mentioned. In addition, as the specified substance to be extracted, for example, nucleic acids such as DNA, RNA and the like can be mentioned.

In the "(b) heating the mixture including the high specific gravity substance, the low specific gravity substance and the gelling agent to dissolve the gelling agent", the gelling agent is dissolved in a liquid which becomes a solvent. The heating condition may be a condition under which the gelling agent is completely dissolved in the liquid or a condition under which the gelling agent is partly dissolved in the liquid, with the proviso that a gel layer is formed by the subsequent centrifugation operation. Solubility of various gelling agents is described for example in "Food polysaccharides - Knowledge on Emulsification, Viscosity Improvement and Gelation (written in Japanese)" edited by Naomichi Kunizaki and Masao Sano (published by Heibunsha, November 25, 2001). In setting the heating conditions, the conventionally known reference can also be referred to. In the case of dissolution of agarose powder in water for example, dissolving state of the gelling agent in the solvent can be verified with the naked eye. Though water suspension of un-dissolved agarose powder is suspended in white, the agarose powder partially dissolved in water can be observed as a transparent particle or a particle in which the central part is white and its surface is transparent (so-called un-dissolved lump of flour). As the heating conditions, though it is not particularly limited, when agarose is used as the gelling agent, for example, the temperature is preferably from 46°C to 120°C, more preferably from 50°C to 110°C, and further preferably from 55°C to 100°C. In addition, the heating time is preferably from 1 minute to 1 hour, more preferably from 2 minutes to 30 minutes, and further preferably from 5 minutes to 20 minutes. In this connection, there is a case in which extraction of the specified substance and removal of the unnecessary substance from the sample can be carried out simultaneously with dissolution of the gelling agent by the heating in this step.

Another embodiment of the solid-liquid separation method of the present invention is a method in which the mixture is obtained by (A) obtaining a solution in which the gelling agent is dissolved; and (B) mixing the solution with the high specific gravity substance and the low specific gravity substance, or with a composition comprising the high specific gravity substance and the low specific gravity substance.

The present invention also includes a composition including a gelling agent capable of forming a thermoreversible gel and a solid substance useful for separating or purifying a desired substance. The composition is preferably used, for example in the aforementioned solid-liquid separation method of the exemplary embodiments of the present invention. The composition preferably contains a hydrophilic gelling agent for forming a thermoreversible gel and an adsorbent carrier. As the adsorbent carrier, an ion exchange resin, activated carbon, silica gel, a chelate resin and an affinity resin can be exemplified, and one selected therefrom may be used as a PCR inhibitor adsorbent resin. The adsorbent carrier is preferably an ion exchange resin.

For example, when the centrifugation method of the exemplary embodiments of the present invention is applied to extracting DNA from a formalin-fixed paraffin-embedded tissue section using a PCR inhibitor adsorbent resin [e.g., extraction of DNA using a commercially available DEXPAT (registered trademark, manufactured by TAKARA BIO INC.)], the composition of the exemplary embodiments of the present invention may contain a hydrophilic gelling agent capable of forming a thermoreversible gel, a PCR inhibitor adsorbent resin (a chelate resin) and a surfactant aqueous solution. When thoroughly suspended aforementioned composition is poured into a container charged with a formalin-fixed paraffin-embedded tissue section, heated at about 100°C for approximately 10 minutes and then centrifuged at a temperature of equal to or lower than the gelation temperature of the gelling agent, the precipitate of the chelate resin is formed in the lowermost layer, and a gel layer thereon, and an aqueous layer containing the DNA extracted from the formalin-fixed paraffin-embedded tissue section in the upper layer. Since the gel layer is formed between the upper layer and the precipitate by the use of said composition, a possibility of causing contamination of the PCR inhibitor adsorbent resin in fractionating the aqueous layer is sharply reduced so that fractionation of the aqueous layer becomes easy. For example, it is possible to collect the aqueous layer by decantation. In the case of the conventional method, upper part of the precipitate of PCR inhibitor adsorbent resin after centrifugation becomes a state of slurry and the PCR inhibitor adsorbent resin is transparent, so that there was a case of causing contamination of the resin by accident in fractionating the aqueous layer. Since there is a possibility that the PCR inhibitor adsorbent resin inhibits the subsequent treatment of the nucleic acid such as PCR, it is desirable to avoid its contamination into the DNA solution as low as possible. From the aqueous layer containing the DNA extracted by said method, substances derived from the formalin-fixed paraffin-embedded tissue section, which inhibits the subsequent treatment of the nucleic acid such as PCR, are removed. In this connection, though it is not particularly limited, when the exemplary embodiments of the present invention are applied to the extraction of DNA from a formalin-fixed paraffin-embedded tissue section using a PCR inhibitor adsorbent resin, agarose is desirable as the hydrophilic gelling agent.

The present invention includes a kit containing a hydrophilic gelling agent capable of forming a thermoreversible gel and an adsorbent carrier. The kit may further comprise, for example, a thermostable polymerase for nucleic acid amplification reaction, a buffer solution, a magnesium salt, a dNTP mixture solution and the like. Examples of the hydrophilic gelling agent capable of forming a thermoreversible gel and the adsorbent carrier include those described above.

An embodiment of the centrifugation method of the present invention is a liquid-liquid separation method. The liquid-liquid separation method of the exemplary embodiments of the present invention is a centrifugation method for separating a liquid mixture into two or more liquid layers based on the difference in specific gravity, using a thermoreversible gel and/or a gelling agent capable of forming a thermoreveresibel gel which forms a layer at a desired position. By the liquid-liquid separation method of the exemplary embodiments of the present invention, contamination of a high specific gravity substance can be prevented in carrying out fractionation of a low specific gravity substance in the upper layer. The liquid-liquid separation method of the exemplary embodiments of the present invention can be used for the separation of two or more liquids which are not mutually mixable. An organic liquid can be suitably exemplified as the above-mentioned high specific gravity substance, and an aqueous liquid such as an aqueous solution can be suitably exemplified as the low specific gravity substance. That is, one of the suitable embodiments of the liquid-liquid separation method is a method which comprises a step for obtaining a mixture containing an organic liquid, an aqueous liquid and a thermoreversible gel and/or a gelling agent capable of forming a thermoreversible gel, and a step for forming an organic layer in the lowermost layer, a gel layer in its upper layer and a aqueous layer in the uppermost layer by subjecting said mixture to centrifugation.

When a step for collecting the low specific gravity substance is included after centrifugation, there is no particular limitation to the collection method, and the liquid layer including the low specific gravity substance can also be collected, for example by decantation.

The organic liquid which can be used in the liquid-liquid separation method of the exemplary embodiments of the present invention is not particularly limited with the proviso that it has a specific gravity of higher than that of water, and for example, phenol, chloroform and isoamyl alcohol can be exemplified, and it may be a mixed liquid containing two more of them. In addition, as the above-mentioned aqueous liquid, a nucleic acid aqueous solution can for example be mentioned. As the above-mentioned thermoreversible gel, there is no particular limitation with the proviso that it forms a gel layer at a desired position as a result of centrifugation, preferably between the high specific gravity substance (for example, an organic liquid) and the low specific gravity substance (for example, an aqueous liquid).

Though it is not particularly limited, the liquid-liquid separation method of the exemplary embodiments of the present invention is useful as a method for removing protein from a nucleic acid aqueous solution, such as phenol treatment, phenol/chloroform treatment, chloroform/isoamyl alcohol treatment and the like. In these protein removing methods, an organic layer is formed in the lower layer after centrifugation, and a layer containing denatured protein in its upper layer and an aqueous layer in the uppermost layer, but it is apt to cause contamination of the denatured protein when collecting the aqueous layer. When the liquid-liquid separation method of the exemplary embodiments of the present invention is applied to the method for removing a protein from a nucleic acid aqueous solution, it is possible to effect formation of a layer of the thermoreversible gel between the layer containing denatured protein and the aqueous layer, so that contamination of the denatured protein into the aqueous layer can be prevented conveniently. In this connection, it is general to use a mixed liquid containing phenol, chloroform and isoamyl alcohol at a volume ratio of 25:24:1 in the phenol/chloroform treatment and it is general to use a mixed liquid containing chloroform and isoamyl alcohol at a volume ratio of 24:1 in the chloroform/isoamyl alcohol treatment. In this connection, in the aforementioned phenol treatment, phenol/chloroform treatment and chloroform/isoamyl alcohol treatment of nucleic acid aqueous solution, a gelling agent gelatinized in advance can be used without dissolving it.

### EXAMPLES

The following illustratively describes the exemplary embodiments of the present invention based on examples, but the present invention is not restricted by these examples.

### Example 1

The centrifugation method which uses a gelling agent was tested by applying it to centrifugation in extracting DNA from rice. In this connection, as the respective reagents to be used in the extraction of DNA from rice, those attached to a rice DNA extraction kit [(polished rice 20 grain scale) (for PCR Kit for rice discrimination). manufactured by TAKARA BIO INC.] were used.

Four suspensions were prepared by adding 4.5 mg, 9 mg, 15 mg or 22.5 mg of SeaKem (registered trademark) GTG Agarose (manufactured by Lonza) to 500 µl of Lysis Buffer A and suspending therein. The thus prepared 4 suspensions were transferred onto a boiling water bath, thereby completely dissolving the agarose. Next, Lysis Buffer B heated in advance on a boiling water bath was added to respective suspensions in 200 µl portions and mixed therewith, and these were kept on the boiling water bath as agarose solutions until their use in the following test.

Five tubes were prepared by putting 20 grains of rice into each of 2 ml capacity microtubes and then adding 540 µl of sterile water, and allowed to stand overnight at room temperature. Lysis Buffer A was added in 300 µl portions to each of the tubes, the rice grains ere pulverized until lumps run out, and Lysis Buffer B was further added in 120 µl portions and mixed therewith. Protease K (manufactured by TAKARA BIO INC.) was added thereto in 10 µl portions and mixed and then immediately heated to 55°C and kept at the same temperature for 1 hour. During this period of time, reversion mixing was carried out after 10 minutes, after 20 minutes and after 40 minutes of the commencement of the heating.

Next, 4 samples were prepared by adding the aforementioned 4 agarose solutions in 200 µl portions. In this connection, a sample was prepared as a control by adding 200 µl of Lysis Buffer mixture (Lysis Buffer A:Lysis Buffer B=5:2) instead of the agarose solution. On these 5 samples, centrifugation was carried out at 4°C and at 17,000 G for 10 minutes.

After centrifugation, whole volume of each supernatant (aqueous layer in the case of samples which formed a gel layer in the lower layer) was collected and weighed. Shown in Table 1 are the agarose concentration of each sample at the time of centrifugation, the condition of each sample after centrifugation and the collection yield of each supernatant (aqueous layer) after centrifugation.

**[Table 1]**

| Agarose concentration (%) | Formation of gel layer | Collection yield of supernatant (aqueous layer) (µl) | Condition after centrifugation |
|---|---|---|---|
| 0 (control) | - | 770 | Fig. 2 |
| 0.15 | Formed | 760 | Fig. 3 |
| 0.30 | Formed | 490 | |
| 0.50 | Formed | 300 | |
| 0.75 | Formed | 20 | |

By adding the agarose solution to each sample before centrifugation and carrying out centrifugation at the agarose gelation temperature or lower, an aqueous layer was formed in the upper layer, and a gel layer in its lower layer and the precipitate of rice pulverization product in the lowermost layer. Since a gel layer was formed between the rice pulverization product and the aqueous layer, it was able to separate the aqueous layer easily without paying attention to the contamination of rice pulverization product. On the other hand, the rice pulverization product was contaminated in the control while collecting the supernatant. In this connection, collection yield of the aqueous layer in the samples to which agarose was added became small in comparison with the control, but a tendency was found that the collection yield of aqueous layer increases as the agarose concentration becomes low.

### Example 2

The precipitation separation method which uses a gelling agent was tested by applying it to centrifugation in extracting DNA from a plant by a benzyl chloride method. In this connection, as the respective reagents to be used in the benzyl chloride method, those which are attached to the Plant DNA Isolation Reagent (manufactured by TAKARA BIO INC.) were used.

Four suspensions were prepared by putting 1 ml of sterile water into each of 1.5 ml capacity microtubes and adding 4.5 mg, 9 mg, 15 mg or 22.5 mg of Seakem (registered trademark) GTG Agarose (manufactured by Lonza) thereto and suspending therein. The thus prepared 4 suspensions were transferred onto a boiling water bath, thereby completely dissolving the agarose. These were kept on the boiling water bath as agarose solutions until their use in the following test.

Spinach was cut into pieces of 3 mm square or less and put into five 1.5 ml capacity microtubes in 50 mg portions, frozen at -20°C and then thawed at room temperature. The spinach was pressed approximately 10 times against the bottom of each microtube with a Pipetman tip to grind the plant tissue. Extraction Solution 1 was added thereto in 400 µl portions and stirred for 5 seconds using Vortex Mixer. After spin down, Extraction Solution 2 was added thereto in 80 µl portions and stirred for 5 seconds using Vortex Mixer. After spin down, Extraction Solution 3 was added thereto in 150 µl portions, stirred for 5 seconds using Vortex Mixer and then spun down again.

Next, 4 samples were prepared by adding the aforementioned agarose solutions having different concentrations thereto in 120 µl portions. In this connection, a sample was also prepared as a control by adding 120 µl of a mixed liquid of Extraction Solutions to which agarose was not added. On these samples, centrifugation was carried out at 4°C and at 17,000 G for 15 minutes.

After centrifugation, whole volume of the upper layer was collected in a 1.5 ml capacity microtube and weighed. A DNA solution was prepared from each of the thus collected upper layers by the subsequent operation in accordance with the standard protocol of Plant DNA Isolation Reagent. Shown in Table 2 are the agarose concentration of each sample at the time of centrifugation, the condition of each sample after centrifugation and the collection yield of each upper layer after centrifugation.

**[Table 2]**

| [Table 2] Agarose concentration (%) | Formation of gel layer | Collection yield of upper layer (µl) | Condition after centrifugation |
|---|---|---|---|
| 0 (control) | - | 550 | Fig. 4 |
| 0.15 | Not formed | 550 | |
| 0.30 | Formed | 500 | Fig. 5 |
| 0.50 | Formed | 280 | |
| 0.75 | Formed | 110 | |

By adding the agarose solution to each sample before centrifugation and carrying out centrifugation at the agarose gelation temperature or lower, it was able to effect formation of an organic layer, a layer of precipitate of the plant tissue ground product, a white layer, a gel layer and an aqueous layer in order from the lowermost layer. In this connection, it is considered that the white layer is a precipitate comprising the plant tissue-derived polysaccharides as the main component. From the samples which formed the gel layer under the aqueous layer, it was able to collect the upper layer without paying attention to the contamination of the further lower layers of the organic layer, precipitate and the like. In the control group on the other hand, it was necessary to pay considerable attention in collecting the upper layer alone, because precipitates (the plant residue and substances of its upper white layer) were whirled up by merely flipping the microtube lightly.

### Example 3

The precipitation separation method which uses a gelling agent was tested by applying it to centrifugation of a reagent for extracting DNA from a formalin-fixed paraffin-embedded tissue section.

Agarose LO3 "TaKaRa" (manufactured by TAKARA BIO INC.) was added to and suspend in DEXPAT (registered trademark, manufactured by TAKARA BIO INC.) which is a mixture of a PCR inhibitor adsorbent resin from a formalin-fixed paraffin-embedded tissue section and a special surfactant solution to be used in the extraction of DNA, to a final concentration of 0.1 %, 0.2%, 0.3 % or 0.4%.

A 500 µl portion of each of the 4 suspensions was transferred into a separate 1.5 ml capacity microtube. In addition, 500 µl of agarose-un-added DEXPAT (registered trademark) put into a 1.5 ml capacity microtube was also prepared as a control.

Each of these 5 suspensions was heated at 100°C for 10 minutes using a heat block and then centrifugation was carried out at 4°C and at 17,000 G for 10 minutes.

After centrifugation, whole volume of each upper layer was collected in a new 1.5 ml capacity microtube and weighed. Shown in Table 3 are the agarose concentration of each sample at the time of centrifugation, the condition of each sample after centrifugation and the collection yield of each upper layer after centrifugation.

**[Table 3]**

| Agarose concentration (%) | Formation of gel layer | Collection yield of upper layer (µl) | Condition after centrifugation |
|---|---|---|---|
| 0 (control) | - | 300 | Fig. 6 |
| 0.1 | Formed | 300 | |
| 0.2 | Formed | 270 | Fig. 7 |
| 0.3 | Formed | 230 | |
| 0.4 | Formed | 230 | |

By centrifuging the agarose-added DEXPAT (registered trademark) at the agarose gelation temperature or lower, it was able to effect formation of a gel layer in intermediate layer between the lowermost layer precipitate (resin layer) and upper layer aqueous layer. In the samples in which the gel layer was formed in the intermediate layer, it was able to collect the upper layer without paying attention to the contamination of the lowermost layer resin. In the control on the other hand, a slurry form precipitate on the upper side of the resin layer was contaminated during collection of the upper layer.

### Example 4

Using centrifugation of a reagent for extracting DNA from a formalin-fixed paraffin-embedded tissue section as a model system, heating condition of a mixture containing agarose powder and a liquid substance was examined.

A solution was prepared by removing the resin from DEXPAT (registered trademark), and SeaKem (registered trademark) GTG Agarose was added thereto to a final concentration of 0.15%. This was dispensed in 500 µl portions into seven 1.5 ml capacity microtubes and heated at 36°C, 40°C, 45°C, 55°C, 65°C, 75°C or 85°C for 10 minutes using a heat block. After the heating, centrifugation was carried out at 4°C and at 17,000 G for 10 minutes, thereby collecting the upper layer. Shown in Table 4 are the condition of each sample after heating, the condition of each sample after centrifugation and the collection yield of each upper layer after centrifugation.

**[Table 4]**

| Tempera-ture (°C) | Condition of sample after heating | Condition of sample after centrifugation | Collection yield of upper layer (µl) |
|---|---|---|---|
| 36 | Agarose powder was not dissolved. | Agarose powder was precipitated, gel layer was not formed. | 480 |
| 40 | Agarose powder was not dissolved. | Agarose powder was precipitated, gel layer was not formed. | 480 |
| 45 | Agarose powder was not dissolved. | Agarose powder was precipitated, gel layer was not formed. | 460 |
| 55 | Hydration and dissolution of agarose powder surface were observed. | Thin gel layer was formed on the upper side of white precipitate. | 440 |
| 65 | Hydration and dissolution of agarose powder surface were observed. | Thin gel layer was formed on the upper side of white precipitate. | 410 |
| 75 | Almost complete dissolution of agarose was found. | Gel layer was formed in the lower layer. | 430 |
| 85 | Agarose was completely dissolved. | Gel layer was formed in the lower layer. | 280 |

From the results of this example, it was revealed that even in the state of partial dissolution of the gelling agent which is not completely dissolved in the liquid, it is possible to effect formation of the gel layer by the subsequent centrifugation.

### Example 5

A case in which centrifugation was carried out after gelatinization of a solid-liquid mixture was tested.

A 500 µl portion of a suspension prepared by suspending SeaKem (registered trademark) GTG Agarose in DEXPAT to a final concentration of 0.15% was put into a 1.5 ml capacity microtube. This was heated at 100°C for 10 minutes using a heat block and then cooled at 4°C until the whole suspension was gelatinized. Thereafter, centrifugation was carried out for 10 minutes under a condition of 4°C and 17,000 G.

As a result, it was able to effect formation of a gel layer between the lowermost layer precipitate (resin layer) and the upper layer aqueous layer and it was able to collect 240 µl of the aqueous layer. By this example, it was revealed that even when the whole solid-liquid mixture is gelatinized after dissolution of the gelling agent, it is possible to carry out solid-liquid separation by centrifugation.

### Example 6

Comparison of the precipitation separation method which uses a gelling agent with a conventional method was carried out by applying them to centrifugation of a reagent for extracting DNA from a formalin-fixed paraffin-embedded tissue section.

DEXPAT (registered trademark) was thoroughly suspended and dispensed in 500 µl portions into two 1.5 ml capacity microtubes (gelling agent-un-added sample). Also, 500 µl of a suspension prepared by adding SeaKem (registered trademark) GTG Agarose to DEXPAT (registered trademark) to a final concentration of 0.15% and suspending therein was pored into a 1.5 ml capacity microtube (gelling agent-added sample).

These suspensions were heated at 100°C for 1 minute using a heat block. A portion of each of the thus heated suspensions was added dropwise to paraffin-embedded rat tissue sections fixed on a slide glass, and then the sections were sliced thinner and respectively collected in the original microtubes charged with the suspensions. Subsequently, each suspension was heated at 100°C for 10 minutes. In this connection, each suspension was reversion-mixed mildly from 2 to 3 times after 5 minutes from the commencement of the heating. After completion of the heating, centrifugation was carried out for 10 minutes under a condition of 4°C and 17,000 G, and after allowing it to stand still on ice for 5 minutes, the supernatant (aqueous layer) was colected. In this connection, in collecting the supernatant, a considerable attention was paid to one of the two gelling agent-un-added samples such that the resin layer was not contaminated into the supernatant, while the resin layer of the other sample was intentionally allowed to contaminate in a small amount into the supernatant.

Using 1 µl or 2.5 µl of each of the thus collected supernatants as the template, PCR was carried out using the rat CCND gene region as the target. The PCR was carried out using Ex Taq (TAKARA BIO INC.) under conditions of a total of 35 cycles, one cycle being 94°C for 30 seconds, 54°C for 60 seconds and then 72°C for 60 seconds, followed by 5 minutes of incubation at 72°C. In this connection, PCR was respectively carried out on two primer sets having different amplification chain lengths (164 bp and 416 bp). The sequences of the primers of the primer set for amplifying 164 bp are shown in SEQ ID NO:1 and SEQ ID NO:2 of SEQUENCE LISTING, and the sequences of the primers of the primer set for amplifying 416 bp in SEQ ID NO:3 and SEQ ID NO:4 of SEQUENCE LISTING, respectively.

As a negative control for the PCR, distilled water was used as a template. As a positive control for the PCR, 1 ng of rat genomic DNA was used as a template.

After completion of the PCR, an 8 µl portion of each reaction liquid was subjected to an agarose gel electrophoresis. A result of the PCR using the 164 bp region in rat CCND gene as the target sequence is shown in Fig. 8, and a result of the PCR using the 416 bp region in rat CCND gene as the target sequence in Fig. 9. As a result, the amplification product was stably obtained when the supernatant collected using the gelling agent-added sample was used as the template (the lane 5 and the lane 8 of Fig. 8 and Fig. 9). On the other hand, among the gelling agent-un-added samples, inhibition of PCR was found when the sample in which the resin layer was intentionally allowed to contaminate in a small amount was used as the template (the lane 4 and the lane 7 of Fig. 8 and Fig. 9). In addition, even in the case of using, as the template, the supernatant collected by paying a considerable attention in order to avoid contamination of the resin layer, there was a case of not finding the amplification (the lane 6 of Fig. 8) and a case of showing a smear-like electrophoresis image (the lane 3 lane of Fig. 9).

As described in the above, while it was difficult to avoid contamination of the precipitate in collecting the supernatant by the conventional method which does not use a gelling agent, it was able by the method of the exemplary embodiments of the present invention to avoid contamination of the precipitate into the supernatant without paying a special attention.

### Example 7

A liquid-liquid separation method using a gelling agent was examined.

A 9 mg portion of SeaKem (registered trademark) GTG Agarose was suspended in 3 ml of sterile distilled water and then heated at 100°C until the agarose powder was completely dissolved. A 750 µl portion of this was poured into a 1.5 ml capacity microtube and then cooled to 4°C, thereby effecting formation of a 0.3% agarose gel. Next, cells were collected from 100 µl of a culture obtained by culturing an *Escherichia coli* strain JM109 at 37°C overnight. The cells were suspended by adding 200 µl of sterile distilled water thereto and then disrupted by 10 minutes of heating at 100°C. Whole volume of this (200 µl) was poured into the aforementioned 1.5 ml capacity microtube in which the 0.3% agarose gel was formed, 200 µl of a phenol/chloroform/isoamyl alcohol (volume ratio = 25:24:1) mixed liquid was added thereto and then vigorously mixed with a hand. Subsequently, centrifugation was carried out under a condition of 17,000 g, room temperature and 10 minutes.

As a result, an organic layer was formed in the lowermost layer and a gel layer was formed between the denatured protein-containing layer which is an upper layer of the organic layer and the uppermost layer aqueous layer. By this, it was able to collect the aqueous layer without paying attention to the contamination of denatured protein.

While the invention has been described in detail and with reference to specific embodiments thereof, it will be apparent to one skilled in the art that various changes and modifications can be made therein without departing from the scope thereof.

In the exemplary embodiments of the centrifugation method of the present invention, the contamination of a high specific gravity substance into a low specific gravity substance, which was a problem of the conventional centrifugation methods, can be easily prevented. Thus, the centrifugation method according to the present invention is useful in a broad range of technical field where centrifugation methods are used.

### SEQUENCE LISTINGS

SEQ ID NO: indicates the primer to amplify the DNA fragment of rat CCND gene. SEQ ID NO:2 indicates the primer to amplify the DNA fragment of rat CCND gene. SEQ ID NO:3 indicates the primer to amplify the DNA fragment of rat CCND gene. SEQ ID NO:4 indicates the primer to amplify the DNA fragment of rat CCND gene.

## Claims

1. A centrifugation method for separating a high specific gravity substance and a low specific gravity substance, which comprises:
centrifuging a mixture comprising a high specific gravity substance, a low specific gravity substance, and at least one of a thermoreversible gel and a dissolved gelling agent capable of forming a thermoreversible gel,
wherein the thermoreversible gel or the dissolved gelling agent forms a gel layer which separates the low specific gravity substance and the high specific gravity substance.

2. The centrifugation method according to claim 1, wherein the centrifugation is carried out at a temperature equal to or lower than the gelation temperature of the gelling agent.

3. The centrifugation method according to claim 1, wherein the mixture comprises a solid and a liquid.

4. The centrifugation method according to claim 1, wherein the mixture is obtained by:
(a) mixing the high specific gravity substance, the low specific gravity substance and the gelling agent, or mixing a composition comprising the high specific gravity substance and the low specific gravity substance with the gelling agent; and
(b) heating the mixture comprising the high specific gravity substance, the low specific gravity substance and the gelling agent to dissolve the gelling agent.

5. The centrifugation method according to claim 1, wherein the mixture is obtained by:
(A) obtaining a solution in which the gelling agent is dissolved; and
(B) mixing the solution with the high specific gravity substance and the low specific gravity substance, or with a composition comprising the high specific gravity substance and the low specific gravity substance.

6. The centrifugation method according to claim 3, wherein the mixture comprises an ion exchange resin.

7. The centrifugation method according to claim 6, wherein the ion exchange resin is a chelate resin.

8. The centrifugation method according to claim 1, wherein the mixture comprises an aqueous liquid and an organic liquid.

9. The centrifugation method according to claim 8, wherein the organic liquid is at least one selected from the group consisting of phenol, chloroform and isoamyl alcohol.

10. The centrifugation method according to claim 8, wherein the aqueous liquid comprises a nucleic acid.

11. The centrifugation method according to claim 1, wherein the gelling agent is a hydrophilic gelling agent.

12. The centrifugation method according to claim 11, wherein the hydrophilic gelling agent is agarose.

13. The centrifugation method according to claim 1, wherein the gelling agent is partly dissolved in the mixture.

14. The centrifugation method according to claim 1, which further comprises collecting the low specific gravity substance.

15. A composition comprising a hydrophilic gelling agent capable of forming a thermoreversible gel and an ion exchange resin.


SEQUENCE LISTING

<110>  TAKARA BIO INC.

<120>  CENTRIFUGATION METHOD

<130>  T1799 EP

<150>  P2010-111134
<151>  2010-05-13

<160>  4

<170>  PatentIn version 3.5

<210>  1
<211>  25
<212>  DNA
<213>  Artificial Sequence

<220>
<223>  Primer to amplify the DNA fragment of rat CCND gene.

<400>  1
gtctgtgagg aacagaagtg tgaag                                          25


<210>  2
<211>  25
<212>  DNA
<213>  Artificial Sequence

<220>
<223>  Primer to amplify the DNA fragment of rat CCND gene.

<400>  2
gcagtcagcg ggatggtctc tttca                                          25


<210>  3
<211>  25
<212>  DNA
<213>  Artificial Sequence

<220>
<223>  Primer to amplify the DNA fragment of rat CCND gene.

<400>  3
attgtctcaa agcctgccag gagca                                          25


<210>  4
<211>  25
<212>  DNA
<213>  Artificial Sequence

<220>
<223>  Primer to amplify the DNA fragment of rat CCND gene.

<400>  4
taatggttcg catatacaag ggatc                                          25